# EUROPEAN PATENT SPECIFICATION

(11) **EP 2 934 520 B1**
(45) Date of publication and mention of the grant of the patent: **16.11.2016**
(21) Application number: 12812220.7
(22) Date of filing: 19.12.2012
(51) Int. Cl.: A61K 31/427, A61K 38/05, A61K 45/06, A61P 17/00

(54) **USE OF PIDOTIMOD TO TREAT ATOPIC DERMATITIS**
VERWENDUNG VON PIDOTIMOD ZUR BEHANDLUNG VON ATOPISCHER DERMATITIS
UTILISATION DU PIDOTIMOD POUR TRAITER LA DERMATITE ATOPIQUE

(43) Date of publication of application: 28.10.2015
(73) Proprietor: Polichem SA, 1526 Luxembourg (LU)
(72) Inventor: MAILLAND, Federico, CH-6900 Lugano (CH); CASERINI, Maurizio, I-22100 Como (IT)
(74) Representative: Pistolesi, Roberto
(86) International application number: PCT/EP2012/076086
(87) International publication number: WO 2014/094839

(56) References cited:
- IT-B- 1 231 723
- GOURGIOTIS DIMITRIOS ET AL: "Immune modulator pidotimod decreases the in vitro expression of CD30 in peripheral blood mononuclear cells of atopic asthmatic and normal children", JOURNAL OF ASTHMA, ASTHMA PUBLICATIONS SOCIETY, OSSINING, NY, US, vol. 41, no. 3, 1 January 2004 (2004-01-01), pages 285-287, XP008164025, ISSN: 0277-0903, DOI: 10.1081/JAS-120026085
- A. BENGTSSON: "The role of CD30 in atopic disease", ALLERGY, vol. 56, no. 7, 1 July 2001 (2001-07-01), pages 593-603, XP55074403, ISSN: 0105-4538, DOI: 10.1034/j.1398-9995.2001.00137.x
- POZZI E ET AL: "Pidotimod in the treatment of patients affected by bacterial exacerbations of chronic bronchitis", 19940101, vol. 44, no. 12A, 1 January 1994 (1994-01-01), pages 1495-1498, XP008097244,
- TARAMELLI D ET AL: "Effects of pidotimod on macrophage functions in methylprednisolone-treated mice.", ARZNEIMITTEL-FORSCHUNG DEC 1994, vol. 44, no. 12A, December 1994 (1994-12), pages 1425-1430, ISSN: 0004-4172
- GIAGULLI CINZIA ET AL: "Pidotimod promotes functional maturation of dendritic cells and displays adjuvant properties at the nasal mucosa level.", INTERNATIONAL IMMUNOPHARMACOLOGY NOV 2009, vol. 9, no. 12, November 2009 (2009-11), pages 1366-1373, ISSN: 1878-1705
- SIMHADRI VIJAYA LAKSHMI ET AL: "A novel role for reciprocal CD30-CD30L signaling in the cross-talk between natural killer and dendritic cells.", BIOLOGICAL CHEMISTRY JAN 2012, vol. 393, no. 1-2, January 2012 (2012-01), pages 101-106, ISSN: 1437-4315

## Description

The present invention is directed to the use of pidotimod, or a physiologically acceptable salt thereof, to treat atopic dermatitis.

### BACKGROUND OF THE INVENTION

Atopic dermatitis is a non-contagious skin disorder characterized by chronically eczematous skin and sometimes intolerable itching. Although atopic dermatitis can appear at any age, it is most common in children and young adults. Symptoms usually abate before the age of 25 and do not affect the patient's general health. About one out of ten babies develop a form of atopic dermatitis called infantile eczema. Characterized by skin that oozes and becomes encrusted, infantile eczema most often occurs on the face and scalp. The condition usually improves before the child's second birthday, and medical attention can keep symptoms in check until that time. When atopic dermatitis develops after infancy, redness, blistering, oozing, and crusting are less pronounced. The patient's sores become dry, turn from red to brownish-gray, and skin may thicken and become scaly. In dark-skinned individuals, this condition can cause the complexion to lighten or darken. Itching associated with this condition is usually worst at night. It can be so intense that patients scratch until their sores bleed, sometimes causing scarring and infection. Atopic dermatitis affects about 3% of the population of the United States, and about 80% of the people who have the condition also have one or more relatives with the same condition or a similar one. Symptoms tend to be most severe in females. Atopic dermatitis can erupt on any part of the skin, and crusted, thickened patches on the fingers, palms, or the soles of the feet can last for years. While allergic reactions often trigger atopic dermatitis, the condition is thought to be the result of an inherited over-active immune system or a genetic defect that causes the skin to loose abnormally large amounts of moisture.

Treatment of atopic dermatitis includes corticosteroid ointment, topic immunosuppressant including tacrolimus or pimecrolimus and emollients. In severe cases that do not respond to other treatments, oral immunosuppressant medications are sometimes prescribed, such as cyclosporine, azathioprine and methotrexate. However, these treatments require patients to take regular blood tests as they can have significant side effects on the kidneys and liver.

Pidotimod, whose chemical name is (4*R*)-3-(5-oxo-L-prolyl)-1,3-thiazolidine-4-carboxylic acid, is a synthetic drug known for its capability to increase the immune response in animal models and in human beings; it was disclosed for the first lime in IT1231723. In vitro studies both from animal and human specimens have documented a good activity on innate and adaptive immune responses and have been confirmed by in vivo clinical studies, demonstrating the efficacy of pidotimod in reducing the rate of recurrent infections of the upper respiratory and urinary tract in children. Same results were obtained in recurrent respiratory tract infections in adults. D. Gourgiotis et al., Journal of Asthma, Vol.41(3), pages 285-287, 2004, describes that the immune modulator pidotimod decreases the in vitro expression of CD30 in peripheral blood mononuclear cells of atopic asthmatic and normal children. More importantly, these effects are more evident in setting of immune defects such as senescence, Down syndrome, surgery, and cancer (Riboldi et al. Int J Immunopathol Pharmacol. 2009; 22(2): 255-62). Due to its capability to stimulate the immune system, pidotimod is believed to worsen those conditions characterized by an increased immune activity and its use is not recommended in such diseases.

Contrary to any expectation, it has now been surprisingly found that pidotimod, besides being active on illnesses characterized by immune defects, may be of benefit in patients with atopic dermatitis, by altenuating the skin lesions typical of such a skin disorder.

### DESCRIPTION OF THIS INVENTION

The object of the present invention is represented by the use of pidotimod, or a physiologically acceptable salt thereof, for use in the treatment of atopic dermatitis.

For the treatment of the present invention, pidotimod, or a physiologically acceptable salt thereof, is preferably administered topically.

When topically administered, pidotimod, or a physiologically acceptable salt thereof, may be in the form of semi-solid or liquid formulations containing pidotimod or a physiologically acceptable salt thereof, together with at least a pharmaceutically acceptable excipient and/or adjuvant; such formulations may be in the form of solutions, emulsions or suspensions, creams, gels and ointments.

Such semi-solid or liquid formulations may have a w/w concentration in pidotimod from 0.1% to 20%, more preferably from 1% to 15%, most preferably from 5% to 10%. They are particularly suitable to treat atopic dermatitis by direct application over the skin lesions.

These pharmaceutical compositions may be prepared according to conventional techniques, may contain pharmaceutically acceptable excipients, adjuvants and/or carriers, and may also contain, in combination, one or more active principles with complementary or, in any case, useful activity.

The active agents which may be used in combination with pidotimod in the treatment of the present invention include, but are not limited to, immunosuppressive agents, Vitamin D and analogues, Vitamin A related compounds, corticosteroids, biologies; such active ingredients may be administered together with pidotimod (i.e. they may be for instance contained in the same composition as pidotimod) or they may be administered separately from or in temporal proximity with pidotimod, either by systemic (oral, intravenous, intramuscular) route or by topical route, directly on the skin or nail lesions.

Examples of immunosuppressive agents include methotrexate, azathioprine, cyclosporine, fumaric acid, tacrolimus or pimecrolimus and corticosteroids; examples of Vitamin D analogues include calcitriol, calcipotriol and tacalcitol; examples of Vitamin A related compounds include retinoids, tretinoine, isotretinoine, etretinate, acitretine, tazarotene, bexarotene and adapalene; examples of biologies include alefacept, etanercept, and monoclonal antibodies adalimumab, infliximab, ustekinumab. Examples of the compositions prepared according to the present invention include: creams, gels, ointments, solutions, emulsions and suspensions for topical application.

The pharmaceutical compositions and the uses of the present invention will now be more fully described by the following examples. It should, however, be noted that such examples are given by way of illustration and not of limitation.

### EXAMPLE 1

An oil in water cream having the following w/w % composition was prepared:

| | | |
|---|---|---|
| 1. | Pidotimod | 10.00% |
| 2. | Tris(hydroxymethy)methylamine* | 5.20% |
| 3. | Lactic Acid | 0.20% |
| 4. | Disodium EDTA | 0.10% |
| 5. | Glycerin | 5.00% |
| 6. | Xanthan Gum | 0.25% |
| 7. | Hydroxypropyl Chitosan | 0.50% |
| 8. | Emulsifiers | 15.50% |
| 9. | Medium chain Triglycerides | 3.00% |
| 10. | 2-Octyldodecyl Alcohol | 2.00% |
| 11. | Diethylene Glycol Monoethyl Ether | 5.00% |
| 12. | DL-Alpha Tocopheryl Acetate | 0.50% |
| 13. | Decamethylcyclopentasiloxane | 3.00% |
| 14. | Preservatives | 1.00% |
| 15. Purified Water | | q.s. to 100.00% |
| * tromethamine | | |

### Preparation

In the main vessel, solubilize components 1, 2, 3, 4, 5 in part of water. Add Xanthan Gum and disperse thoroughly until homogeneity. Separately solubilize component 7 in part of water, then add it to the main vessel while stirring. Heat the phase at 70 - 75°C. In another vessel combine the components 8, 9, 10, 11, 12 and heat at 70 - 75°C while stirring. Combine the two phases heated at the same temperature and homogenize for about 10 minutes. Cool down to 40° and add on sequence components 13 and 14, homogenizing after each addition.
Cool down to room temperature under moderate stirring.

### EXAMPLE 2

A topical solution having the following w/w % composition was prepared:

| | | |
|---|---|---|
| 1. | Pidotimod | 10.00% |
| 2. | Tris(hydroxymethy)methylamine | 5.00% |
| 3. | Disodium EDTA | 0.10% |
| 4. | Propylene Glycol | 5.00% |
| 5. | Lactic acid | 0.15% |
| 6. | Hydroxypropyl Chitosm | 1.00% |
| 7. | Purified water | q.s. to 100.00% |

### Preparation

Solubilize components 1, 2, 3, 4, 6 in water. Add component 7 and mix until clear solution is obtained.

### EXAMPLE 3

A detergent body and scalp formulation having the following w/w % composition was prepared:

| | | |
|---|---|---|
| 1. | Pidotimod | 5.00% |
| 2. | Tris(hydroxymethyl)methylamme | 2.50% |
| 3. | Purified water | q.s to 100.00% |
| 4. | Hydroxypropyl Chitosan | 1.500% |
| 5. | Surfactants | 43.00% |
| 6. | Citric Acid Monohydrate | 0.30% |
| 7. | Sodium Chloride | 1.00% |
| 8. | Benzyl alcohol | 1.00% |
| 9. | Diethyleneglycol Lauryl Ether | 2.00% |

### Preparation

In the main vessel combine the surfactant mixture 5. Add component 8 and solubilize until clear solution. Add component 9 and mix until homogeneity. Separately, in part of water, solubilize components 1, 2, 4, 6 and add it in the main vessel while stirring. Finally regulate viscosity adding component 7. Mix until clear solution.

### EXAMPLE 4

A topical gel formulation having the following w/w % composition was prepared:

| | | |
|---|---|---|
| 1. | Purified water | q.s to 100.00% |
| 2. | Pidotimod | 10.00% |
| 3. | Tris(hydroxymethyl)methylamine | 5.00% |
| 4. | Disodium Edta | 0.10% |
| 5. | Glycerin | 5.00% |
| 6. | 5-Ureidohydantoin | 0.30% |
| 7. | Thickeners | 0.80% |
| 8. | Hydroxypropyl Chitosan | 0.50% |
| 9. | Preservatives | 0.33% |

### Preparation

In the main vessel combine the components 1, 2, 3, 4, 5, 6, and 9. Mix until clear solution. Add thickeners homogenizing after each addition and until fully dispersed. Separately solubilize component 8 in part of water and add it in the main vessel while stirring. Mix until homogeneity.

### EXAMPLE 5

An evaluation of the activity of pidotimod was tested on patients affected by atopic dermatitis, in order to assess the efficacy in terms of improvement of the pathology by means of erythema evaluation. The safety of the treatment was also evaluated. The study was performed in 5 patients (4 females and 1 male, aged between 22 - 35 years, mean = 29) with a clinical diagnosis of atopic dermatitis having as inclusion criteria the affection of the anterior flexural crease of the elbow or of the knee. The patients, before and during pidotimod treatment, did not take any concomitant treatment with local corticosteroids or any systemic therapy.
The study product was taken with the composition of the Example 1 at a dosage of two applications daily on the affected skin.

During the trial, the following visits were performed:
- baseline - T0 (before the product use)
- intermediate visit - T6 (after 6 weeks of treatment)
- final visit - T12 (after 12 weeks of treatment)

No relevant event, which may have interfered to the test results, occurred during the study period.
The efficacy of the product was expressed by mean of a 5 points erythema score, assessing the score at the baseline, at the intermediate visit and the final one. The results were reported in the table below:

| **Age/Sex (M, F)** | **Localization** | **History of Atopic Dermatitis** | **Concomitant Treatments** | **Erythema Score** | | |
|---|---|---|---|---|---|---|
| | | | | **Baseline Mean: 2.8 SD: 0.84** | **6 weeks Mean: 2.4 SD: 1.34** | **12 weeks Mean: 1.2 SD: 1.30** |
| 22 F | inner side of the elbow | 12 yrs | moisturizer | 2 | 1 | 0 |
| 31 M | inner side of the knee | 16 yrs | sodium hyaluronate | 4 | 4 | 3 |
| 28 F | inner side of the elbow | 12 yrs | moisturizer | 3 | 3 | 2 |
| 35 F | inner side of the elbow | 20 yrs | - | 2 | 1 | 0 |
| 29 F | inner side of the elbow | 9 yrs | moisturizer | 3 | 3 | 1 |

The mean value of the erythema score at baseline was 2.8 with a standard deviation of 0.84; at the intermediate visit, the mean of the erythema score was 2.4 (SD = 1.34), while at the end of treatment the mean of erythema score values was 1.2 (SD = 1.30). The obtained results showed that the study product determined a statistically significant increase (Student t test p < 0.05) of the erythema score value at T12 vs. T0, while there is not a statistically significance at T6 vs. T0.

It is important to highlight that the improvement in the clinical evidences of the pathology, has been shown in all patients, with a better effect in patients with a mild-moderate erythema.
Moreover, the treatment was very well tolerated and no side effects were reported.
In conclusions, the treatment with pidotimod (800mg/die) was able to improve the erythema score, identified as index needed to measure the severity of atopic dermatitis with a result, at the end of the treatment, lasted 12 weeks, statistically significant (Student t test p < 0.05), compared to the baseline that suggests the use of pidotimod in the treatment of atopic dermatitis, mild to moderate.

## Claims

1. Pidotimod or a physiologically acceptable salt thereof, for use in the treatment of atopic dermatitis.

2. Pidotimod or a physiologically acceptable salt thereof for use according to claim 1, **characterized in that** it is administered to a human.

3. Pidotimod or a physiologically acceptable salt thereof for use according to claim 1, **characterized in that** it is administered topically.

4. Pidotimod or a physiologically acceptable salt thereof for use according to claim 3, **characterized in that** it is administered by means of a semi-solid or liquid formulation.

5. Pidotimod or a physiologically acceptable salt thereof for use according to claim 4, **characterized in that** semi-solid formulation is a cream, a gel, an ointment or an emulsion.

6. Pidotimod or a physiologically acceptable salt thereof for use according to claim 4, **characterized in that** said liquid formulation is a solution or a suspension.

7. Pidotimod or a physiologically acceptable salt thereof for use according to claim 4, **characterized in that** said formulation has a w/w concentration in pidotimod or a salt thereof from 0.1% to 20%, preferably from 1% to 15%, more preferably from 5% to 10%.

8. Pidotimod or a physiologically acceptable salt thereof for use according to any of the preceding claims, **characterized in that** it is administered in combination or in temporal proximity with at least one additional active principle.

9. Pidotimod or a physiologically acceptable salt thereof for use according to claim 8, **characterized in that** said at least one additional active principle is selected from immunosuppressive agents, Vitamin D and analogues, Vitamin A related compounds, corticosteroids, biologics.

10. Pidotimod or a physiologically acceptable salt thereof for use according to claim 9, **characterized in that** said at least one immunosuppressive agent is selected from:
methotrexate, azathioprine, cyclosporine, fumaric acid, tacrolimus or pimecrolimus and corticosteroids.

11. Pidotimod or a physiologically acceptable salt thereof for use according to claim 9, **characterized in that** said at least one Vitamin D analogue is selected from calcitriol, calcipotriol and tacalcitol.

12. Pidotimod or a physiologically acceptable salt thereof for use according to claim 9, **characterized in that** said at least one Vitamin A related compound is selected from retinoids, tretinoine, isotretinoine, etretinate, acitretine, tazarotene, bexarotene and adapalene.

13. Pidotimod or a physiologically acceptable salt thereof for use according to claim 9, **characterized in that** said at least one biologic is selected from alefacept, etanercept, and monoclonal antibodies adalimumab, infliximab, ustekinumab.

## Patentansprüche

1. Pidotimod oder ein physiologisch akzeptables Salz davon zur Verwendung bei der Behandlung von atopischer Dermatitis.

2. Pidotimod oder ein physiologisch akzeptables Salz davon zur Verwendung gemäß Anspruch 1, **dadurch gekennzeichnet, dass** es einem Menschen verabreicht wird.

3. Pidotimod oder ein physiologisch akzeptables Salz davon zur Verwendung gemäß Anspruch 1, **dadurch gekennzeichnet, dass** es topisch verabreicht wird.

4. Pidotimod oder ein physiologisch akzeptables Salz davon zur Verwendung gemäß Anspruch 3, **dadurch gekennzeichnet, dass** es mittels einer semi-soliden oder flüssigen Formulierung verabreicht wird.

5. Pidotimod oder ein physiologisch akzeptables Salz davon zur Verwendung gemäß Anspruch 4, **dadurch gekennzeichnet, dass** die semi-solide Formulierung eine Creme, ein Gel, eine Salbe oder eine Emulsion ist.

6. Pidotimod oder ein physiologisch akzeptables Salz davon zur Verwendung gemäß Anspruch 4, **dadurch gekennzeichnet, dass** die flüssige Formulierung eine Lösung oder eine Suspension ist.

7. Pidotimod oder ein physiologisch akzeptables Salz davon zur Verwendung gemäß Anspruch 4, **dadurch gekennzeichnet, dass** diese Formulierung eine Massenkonzentration (w/w) von Pidotimod oder einem Salz davon von 0,1% bis 20%, bevorzugt von 1% bis 15%, mehr bevorzugt von 5% bis 10%, hat.

8. Pidotimod oder ein physiologisch akzeptables Salz davon zur Verwendung gemäß irgendeinem der vorhergehenden Ansprüche, **dadurch gekennzeichnet, dass** es in Kombination oder in zeitlicher Nähe mit mindestens einem zusätzlichen Wirkprinzip verabreicht wird.

9. Pidotimod oder ein physiologisch akzeptables Salz davon zur Verwendung gemäß Anspruch 8, **dadurch gekennzeichnet, dass** dieses mindestens eine zusätzliche Wirkprinzip ausgewählt ist aus immunsuppressiven Agenzien, Vitamin D und Analoga, mit Vitamin A in Beziehung stehende Verbindungen, Corticosteroide, biologische Präparate.

10. Pidotimod oder ein physiologisch akzeptables Salz davon zur Verwendung gemäß Anspruch 9, **dadurch gekennzeichnet, dass** das mindestens eine immunsuppressive Agens ausgewählt ist aus: Methotrexat, Azathioprin, Cyclosporin, Fumarsäure, Tacrolimus oder Pimecrolimus und Corticosteroide.

11. Pidotimod oder ein physiologisch akzeptables Salz davon zur Verwendung gemäß Anspruch 9, **dadurch gekennzeichnet, dass** das mindestens eine Vitamin D-Analog ausgewählt ist aus Calcitriol, Calcipotriol und Tacalzitol.

12. Pidotimod oder ein physiologisch akzeptables Salz davon zur Verwendung gemäß Anspruch 9, **dadurch gekennzeichnet, dass** die mindestens eine mit Vitamin A in Beziehung stehende Verbindung ausgewählt ist aus Retinoid, Tretinoin, Isotretinoin, Etretinat, Acitretin, Tazaroten, Bexaroten und Adapalen.

13. Pidotimod oder ein physiologisch akzeptables Salz davon zur Verwendung gemäß Anspruch 9, **dadurch gekennzeichnet, dass** das mindestens eine biologische Präparat ausgewählt ist aus Alefacept, Etanercept und monoklonale Antikörper Adalimumab, Infliximab, Ustekinumab.

## Revendications

1. Pidotimod ou un sel physiologiquement acceptable de celui-ci, pour une utilisation dans le traitement d'une dermatite atopique.

2. Pidotimod ou un sel physiologiquement acceptable de celui-ci pour une utilisation selon la revendication 1, **caractérisé en ce qu'**il est administré à un humain.

3. Pidotimod ou un sel physiologiquement acceptable de celui-ci pour une utilisation selon la revendication 1, **caractérisé en ce qu'**il est administré par voie topique.

4. Pidotimod ou un sel physiologiquement acceptable de celui-ci pour une utilisation selon la revendication 3, **caractérisé en ce qu'**il est administré au moyen d'une formulation liquide ou semi-solide.

5. Pidotimod ou un sel physiologiquement acceptable de celui-ci pour une utilisation selon la revendication 4, **caractérisé en ce que** la formulation semi-solide est une crème, un gel, une pommade ou une émulsion.

6. Pidotimod ou un sel physiologiquement acceptable de celui-ci pour une utilisation selon la revendication 4, **caractérisé en ce que** ladite formulation liquide est une solution ou une suspension.

7. Pidotimod ou un sel physiologiquement acceptable de celui-ci pour une utilisation selon la revendication 4, **caractérisé en ce que** ladite formulation a une concentration en poids/poids de pidotimod ou d'un sel de celui-ci de 0,1 % à 20 %, de préférence de 1 % à 15 %, mieux encore de 5 % à 10 %.

8. Pidotimod ou un sel physiologiquement acceptable de celui-ci pour une utilisation selon l'une quelconque des revendications précédentes, **caractérisé en ce qu'**il est administré en combinaison ou à proximité temporelle avec au moins un principe actif additionnel.

9. Pidotimod ou un sel physiologiquement acceptable de celui-ci pour une utilisation selon la revendication 8, **caractérisé en ce que** ledit au moins un principe actif additionnel est choisi parmi les agents immunodépresseurs, la vitamine D et ses analogues, les composés apparentés à la vitamine A, les corticostéroïdes, les agents biologiques.

10. Pidotimod ou un sel physiologiquement acceptable de celui-ci pour une utilisation selon la revendication 9, **caractérisé en ce que** l'au moins un agent immunodépresseur est choisi parmi le méthotrexate, l'azathioprine, la cyclosporine, l'acide fumarique, le tacrolimus ou pimécrolimus, et les corticostéroïdes.

11. Pidotimod ou un sel physiologiquement acceptable de celui-ci pour une utilisation selon la revendication 9, **caractérisé en ce que** ledit au moins un analogue de vitamine D est choisi parmi le calcitriol, le calcipotriol et le tacalcitol.

12. Pidotimod ou un sel physiologiquement acceptable de celui-ci pour une utilisation selon la revendication 9, **caractérisé en ce que** ledit au moins un composé apparenté à la vitamine A est choisi parmi les rétinoïdes, la trétinoïne, l'isotrétinoïne, l'étrétinate, l'acitrétine, le tazarotène, le bexarotène et l'adapalène.

13. Pidotimod ou un sel physiologiquement acceptable de celui-ci pour une utilisation selon la revendication 9, **caractérisé en ce que** ledit au moins un agent biologique est choisi parmi l'aléfacept, l'étanercept, et les anticorps monoclonaux adalimumab, infliximab, ustékinumab.
